(19) Europäisches Patentamt
European Patent Office
Office européen des brevets

(11) Publication number: **0 187 049 B1**

(12) # EUROPEAN PATENT SPECIFICATION

(45) Date of publication of patent specification: **23.11.89**

(21) Application number: **85309513.1**

(22) Date of filing: **24.12.85**

(51) Int. Cl.⁴: **C 07 H 17/08**, A 61 K 31/70, C 12 P 19/62 // (C12P19/62, C12R1:29)

(54) **Micromonospora microorganisms and macrolide antibiotic production therewith.**

(30) Priority: **24.12.84 JP 272570/84**

(43) Date of publication of application:
**09.07.86 Bulletin 86/28**

(45) Publication of the grant of the patent:
**23.11.89 Bulletin 89/47**

(84) Designated Contracting States:
**AT BE CH DE FR GB IT LI NL SE**

(56) References cited:
**THE JOURNAL OF ANTIBIOTICS, vol. XXIX, no. 11, November 1976, pages 1171-1181; T. KISHI et al.: "Studies on juvenimicin, a new antibiotic. II Isolation, chemical characterization and structures"**

**THE JOURNAL OF ANTIBIOTICS, vol. XXX, no. 6, June 1977, pages 450-454; A. KINUMAKI et al.: "Macrolide antibiotics M-4365 produced by Micromonospora. II. Chemical structures"**

(73) Proprietor: **YAMANOUCHI PHARMACEUTICAL CO. LTD.**
**No. 3-11 Nihonbashi-Honcho, 2-chome Chuo-ku**
**Tokyo (JP)**

(72) Inventor: **Imai, Harumitsu**
**No. 599, Nakahara-ku Kizuki**
**Kawasaki-shi Kanagawa (JP)**
Inventor: **Suzuki, Ken-Ichi**
**No. 1-67, Futatsuy**
**Kitamoto-shi Saitama (JP)**
Inventor: **Miyazaki, Shigeru**
**No. 1-25, Fujimi-cho**
**Kohnosu-shi Saitama (JP)**
Inventor: **Kadota, Shigenobu**
**No. 1-18-2-608, Higashi juju Kita-ku**
**Tokyo (JP)**

(74) Representative: **Geering, Keith Edwin et al**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 187 049 B1

(56) References cited:
THE JOURNAL OF ANTIBIOTICS, vol. XXXV, no. 2, February 1982, pages 251-253, Tokyo, JP; R.W. VAUGHAN et al.: "Isolation and characterization of two 16-membered lactones: 20-deoxorosaramicin and 20-deoxo-12,13-desepoxy-12,13-dehydrorosaramicin aglycones, from a mutant strain of Micromonospora Rosaria"

THE JOURNAL OF ANTIBIOTICS, vol. XXXIV, no. 8, August 1981, pages 1075-1077, Tokyo, JP; "Mycinamicins, new macrolide antbiotics. V. Isolation and structures of new 16-membered aglycones, mycinolide IV and protomycinolide IV"

## Description

The present invention relates to microorganisms belonging to the novel species *Micromonospora* YS-02930. These microorganisms are capable of producing antibiotics of the macrolide type shown by the following formula:

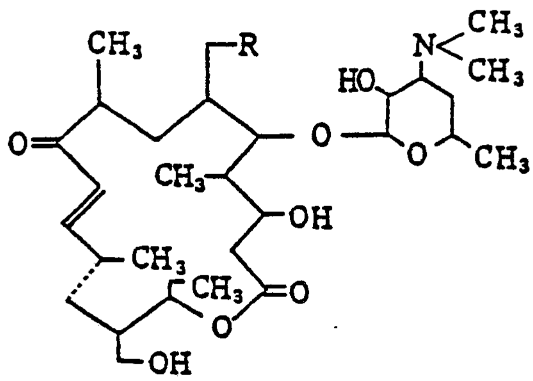

wherein R represents a hydrogen atom or a formyl group and the dotted line denotes a double bond or

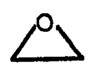

(and in particular capable of producing one, two or all three of such antibiotics YS-02930 K-D, -E and -H) and to a process for producing such antibiotic(s) which comprises culturing such microorganisms and harvesting the antibiotic(s) from the culture solution.

YS-02930 K-D is a compound wherein R denotes a formyl group and the dotted line is a double bond, YS-02930 K-E is a compound wherein R is a formyl group and the dotted line is

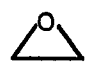

and YS-02930 K-H is a compound wherein R is a hydrogen atom and the dotted line is a double bond, these being referred to hereafter as D-, E- and H-substance, respectively.

The D substance is included in the invention disclosed in U.S. Patent 4,438,109 and the E and H substances in U.S. Patent 4,477,443; these substances are all known compounds. According to the former U.S. patent, the D substance is produced by a chemical synthesis process which comprises using a starting material 3,23-O-methoxymethyl- (or tetrahydrofuranyl)mycaminosyl tylonolide diethylacetal, deoxygenating at the 4′-position thereof and then deblocking protective groups for the hydroxy groups at the 3- and 23-positions and the aldehyde protective group at the 18-position thereof. Further according to the latter U.S. patent, the E substance is chemically synthesized by a process which comprises treating 4′-deoxymycaminosyl tylonolide diethylacetal with a peracid, then epoxygenating the 12- and 13-positions and then deblocking the aldehyde protective group; further the H substance is chemically synthesized by reacting chlorotris(triphenylphosphine)rhodium with 4′-deoxymycaminosyl tylonolide.

However, it has not been known hitherto to produce the aforesaid antibiotics by fermentation.

Some macrolide antibiotics, which differ from those mentioned above, have previously been obtained by biosynthetic fermentation processes.

In the Journal of Antibiotics, Vol. 35, (1982), pages 251 to 253 there is disclosed the isolation and characterisation of two 16-membered lactones, namely 20-Deoxorosaramicin and 20-Deoxo-12,13-desepoxy-12,13-dehydrorosamicin aglycones, from a mutant strain of *Micromonospora* rosaria.

In the Journal of Antibiotics, Vol. 34, (1981), pages 1075 to 1077 there is disclosed the isolation and characterisation of two further 16-membered lactone aglycones, namely mycinolide IV and protomycinolide IV, from the culture filtrate of *Micromonospora* griseorubida sp. nov.

In the Journal of Antibiotics, Vol. 30, (1977), pages 450 to 454, there is disclosed the characterisation of a series of six basic 16-membered lactones designated M-4365 $A_1$, $A_2$, $A_3$, $G_1$, $G_2$ and $G_3$ produced by *Micromonospora* Capillata. M-4365 $A_2$, $A_3$ and $G_3$ were identical with rosamicin, juvenimicins $A_4$ and $B_1$ respectively.

In the Journal of Antibiotics, Vol. 29, (1976), pages 1171 to 1181, there is disclosed the isolation and partial characterisation of a series of eight components, namely juvenimicin $A_1$ to $A_4$ and $B_1$ to $B_4$, from the culture filtrate of *Micromonospora* Chalcea var, izumensis. Juvenimicin $A_3$ was found to be identical with rosamicin.

The present invention provides microorganisms of the novel species YS-20930 belonging to the genus *Micromonospora* capable of producing antibiotic(s) of the above formula. It also provides processes for producing the antibiotics by culturing such microorganisms belonging to the new *Micromonospora* species and harvesting the resulting antibiotic(s).

We have found microorganisms, isolated from soil in conventional manner, that are novel Actinomycetes belonging to the genus *Micromonospora* according to their taxonomical characteristics and that can produce one or two, or all of the D, E and H substances.

A specific example of a microorganism according to the invention is *Micromonospora* sp. YS-02930 K which is a type strain of the species YS-02930.

The species and strain designations YS-02930 and YS-02930 K used herein are our temporary informal nomenclature pending allocation of formal permanent names.

Bacteriological properties of the type strain YS-02930 K, are as follows:

1. Morphological characteristics

No true aerial mycelium is formed on various agar media conventionally used. Microscopic observations reveal that single (rarely 2) spore(s) is/are formed at the tips of sporophores (0.2 to 0.8 μm) branching from the substrate mycelia and formed all around the whole mycelia.

According to the classification by Leudemann et al. [Antimicrob. Ag. Chemoth., 1964, 47—52 (1965)], the sporophores are formed by the Monopodial system.

The spores are spherical (about 0.8—1.2 μm in diameter) and with a smooth surface under electron microscopic observation.

When cultured in liquid medium, hyphae are scarcely branched but longitudinally extended; and a spherical structure (8 to 10 μm in diameter) is observed between hyphae. Electron microscopic observation reveals that these structures are formed by fusion of the hyphae.

2. Cultural characteristics on various media

Cultural characteristics on various media are as shown below.

Unless otherwise indicated, the properties are observed in a conventional manner after culturing at 28°C for 21 days. The color indications are given according to the classification in Color Harmony Manual (Color Research Laboratories, Japan).

| Medium | | Growth Condition |
|---|---|---|
| Starch (1%) yeast extract (0.2%) agar medium | G | good, dim yellow orange |
| | R | pale yellow brown ~ dim yellow orange |
| | S | none |
| Glucose-asparagine agar medium | G | good, yellow brown |
| | R | pale yellow brown ~ yellow brown |
| | S | none |
| Glycerine-asparagine agar medium (ISP-5) | G | no good, brown white |
| | R | colorless — brown |
| | S | none |
| Starch inorganic salts agar medium (ISP-4) | G | somewhat poor, pale yellow brown |
| | R | pale yellow orange ~ pale yellow brown |
| | S | none |

| Medium | | Growth Condition |
|---|---|---|
| Tyrosine agar medium (ISP-7) | G | medium, brown white yellow brown |
| | R | colorless ~ brown white |
| | S | none |
| Nutrient agar medium | G | good |
| Yeast maltose agar (ISP-2) | G | good, dim yellow orange ~ yellow brown |
| | R | dim yellow orange ~ dark yellow orange |
| | S | none |
| Oatmeal agar medium (ISP-3) | G | good, grayish yellow brown |
| | R | dim yellow orange |
| | S | none |
| Bennett agar medium | G | good, dim yellow orange ~ yellow brown |
| | R | dim yellow orange ~ dark yellow orange |
| | S | none |
| Peptone-yeast-iron agar medium (ISP-6) | G | medium, yellow orange |
| | R | yellow orange ~ dark yellow orange |
| | S | none |

(Notes)
G: condition of growth; colour of bacterioflora at the colony surface
R: color of back surface
S: soluble pigment

3. Physiological characteristics:

| | | |
|---|---|---|
| 1) | Growth temperature range | 24—40°C |
| | Optimum growth temperature | 30—33°C |
| 2) | Liquefaction of gelatin gelatin (20°C) | weakly positive |
| | glucose-peptone-gelatin (28°) | weakly positive |
| 3) | Coagulation of skimmed milk | positive |
| | Peptonization of skimmed milk | positive |
| 4) | Nitrate reduction | negative |
| 5) | Starch hydrolysis | negative |

| | | |
|---|---|---|
| 6) | Formation of melanoid pigment<br>tyrosin agar | negative |
| | peptone-yeast-iron agar | negative |

Note: The growth temperature indicates the result of observation from 7 to 21 Days at each temperature (5, 10, 15, 20, 25, 28, 30, 33, 37, 40, 45 and 50°C). The action on milk indicates the results of observation at 37°C from 3 to 21 Days. Others indicate the results of observation at 28°C after 2 weeks, unless otherwise indicated.

4. Assimilation of carbon sources (Pridham-Gottlieb agar medium, cultured at 28°C)

| | | | |
|---|---|---|---|
| Trehalose | − | D-Mannitol | − |
| D-Fructose | − | D-Galactose | − |
| D-Glucose | ± | Maltose | − |
| L-Arabinose | + | Lactose | − |
| D-Xylose | − | D-Sorbitol | − |
| Sucrose | + | Salicin | − |
| Inositol | − | Glycerine | − |
| L-Ramnose | − | α-Melibiose | ∓ |
| Raffinose | − | Soluble starch | + |

(Notes): +: growth
±: slight growth
∓: scarcely any growth
−: no growth.

5. Analysis of cell wall composition:

According to the Lechevalier et al method (Lechevalier, M.P. et al; pp. 227—228 in Dietz, A. et al ed., Actimonycete Taxonomy, SIM Special publication No. 6, 1980), the cell wall components of the strain and acid hydrolysates of the whole cell were analyzed. As a result, it was confirmed that it contained meso-diaminopimelic acid, 3-hydroxydiaminopimelic acid and glycine as characteristic amino acids and as sugar components, xylose and arabinose.

From the foregoing it is seen that the strain YS-02930 K does not form any true mycelium on various agar media but forms a single (rarely 2) spore(s) on sporophores (Monopodial type) branching from substrate mycelium. In liquid culture, the hyphae are scarcely branched but longitudinally extended. The hyphae are occasionally fused to form spherical structures. By analysis of the cell wall and acid hydrolysates of the whole cell, it was confirmed that it contained meso-diaminopimelic acid and glucine as characteristic amino acids, and as sugars, xylose and arabinose. From the foregoing characteristics, this strain is considered to belong to the genus *Micoromonospora.* From survey of known Actinomycetes similar to this strain by Bergey's Manual of Determinative Bacteriology, 8th Edition (1974) and various publications, this strain resembles Micromonospora chalcea and Micromonospora halophytica in that spores are spherical with smooth surface and the color tone grown on agar media is orange to yellow brown. However, as shown in Table 1, the strain YS-02930 K differs from *M. chalcea* in utilization of carbon sources. *M. chalcea* can utilize α-melibiose, raffinose, L-arabinose, D-glucose, D-fructose, D-galactose, starch, sucrose and D-xylose; whereas the strain YS-02930 K can utilize L-arabinose, D-glucose, sucrose and starch but cannot utilize carbon sources other than those described above (it can slightly utilize α-milibiose). Further the strain YS-02930 K has no capability of decomposing cellulose as is noted with *M. chalcea.*

Further, *M. halophytica* can utilize L-arabinose, D-galactose, D-glucose, D-fructose, α-melibiose, raffinose, starch, sucrose and D-xylose as carbon sources, as shown in Table 1, and is greatly different from the strain YS-02930 K in utilization of carbon sources. Further, *M. halophytica* differs from the strain YS-02930 K both in reduction of nitrate (positive) and in decomposition of cellulose (positive).

In addition *Micromonospora carbonacea* is similar to the strain YS-02930 in that spores are spherical with a smooth surface and non-branched long mycelia are formed in liquid culture; and the color tone grown on agar medium is orange ~ yellow brown ~ black. However, with respect to utilization of carbon

sources, *M. carbonacea* is different from the strain YS-02930 K, as shown in Table 1, in utilization of D-xylose, D-fructose and α-melibiose; and also different in reduction of nitrate. Furthermore, the sporophore formation is Sympodial type with *M. carbonacea* but Monopodial type with the strain YS-02930 K.

TABLE 1
Comparison of Utilization of Carbon Sources
(according to the method of PRIDHAM and GOTTLIEB: J. Bacteriol., *56*, 107, 1948)

| | YS-02930K | *M. carbonacea* NRRL 2972 | *M. chalcea* ATCC 12452 | *M. halophytica* NRRL 3097 |
|---|---|---|---|---|
| L-Arabinose | + | + | + | + |
| D-Xylose | − | + | + | + |
| D-Glucose | ± | + | + | + |
| D-Fructose | − | + | + | + |
| Sucrose | + | + | + | + |
| Inositol | − | − | − | − |
| L-Ramnose | − | − | − | − |
| Raffinose | − | − | + | + |
| D-Galactose | − | + | + | − |
| D-Mannitol | − | − | ∓ | − |
| Soluble Starch | + | + | + | + |
| α-Melibiose | ∓ | + | + | + |

+: utilization; ±: poor utilization; ∓: scarcely any utilization; −: no utilization.

In addition, other species belonging to the genus *Micromonospora*, for which the production of macrolide antibiotics has been reported, included (1) *Micromonospora rosaria*, (2) *Micromonospora megalomiciae*, (3) *Micromonospora inositola* and (4) *Micromonospora chalcea* var. *izumensis;* however, (1) produces a soluble pigment of wine red color and shows a characteristic echinulate structure at the spore surface, (2) grows well on tyrosine agar medium and formation of melanoid is noted; (3) does not grow on glucose asparagine agar and utilizes only inositol as a carbon source; and (4) has better utilization of carbon sources as compared to the strain YS-02930 K- especially, the utilization of α-melibiose and raffinose is noted. From the foregoing points, the above-described 4 species are obviously different from the strain YS-02930 K. From these results, strain YS-02930 K is not identical with the previously described species belonging to the genus *Micromonospora.* Therefore, YS-02930 is considered to be a novel species of Micromonospora, and the type strain *Micromonospora* sp. YS-02930 K has been deposited in Fermentation Research Institute, Agency of Industrial Science and Technology, Ministry of International Trade and Industry of Japan under the accession numbers FERM-P No. 7961 (deposited on November 29, 1984) and FERM-BP No. 1076 (June 11, 1986).

Clearly, other microorganisms of genus *Micromonospora* having the characteristic bacteriological properties of strain YS-02930 K belong to the novel species designated YS-02930.

The microorganisms in accordance with the present invention are characterised by producing antibiotic(s) of the above general formula, and some in addition by their taxonomical characteristics described above. Strains of the present invention are susceptible to artificially caused or spontaneous variation, as is observed in other Actinomycetes.

The invention includes strains isolated from the natural environment, strains derived by artificial variation by means of UV rays, X rays, chemicals, etc. and spontaneous variants (e.g. mutants and derivatives of type-strain YS-02930 K).

The microorganisms of the present invention are obtainable by isolation from natural soil but can easily be accessible by restoring the freeze dried bacteria deposited in the Fermentation Research Institute described above.

The microorganisms in accordance with the present invention are preferably cultured in media comprising nutrient sources which the microorganisms utilize. The media may be any of synthetic, semisynthetic or natural, solid or liquid media, but liquid media comprising natural nutrient sources are generally preferred. As the nutrient sources added to the media, any carbon sources may be used as long as they are carbonaceous compounds capable of assimilation;
examples include arabinose, sucrose, starch, glucose, starch, dextrin, palm oil, soybean oil, α-melibios, etc., which may be used singly or in combination. Further, alcohols, organic acids and the like may also be used.

As inorganic and organic nitrogen sources, there may be used, singly or in combination, ammonium chloride, sodium nitrate, ammonium sulfate, ammonium nitrate, urea, etc., peptone, yeast extract, dry yeast, meat extract, gluten meal, corn steep liquor, soybean powder, fish powder, peanut powder, cotton seed meal, casamino acid, various amino acids (e.g., glutamic acid, alanine, lysine, etc.) and the like.

The media may also be supplemented with sulfates, nitrates, chlorides, carbonates, phosphates, etc. of metals such as sodium, potassium, magnesium, calcium, zinc, iron, cobalt, etc., if necessary or desired.

It is preferred that culture be carried out under aerobic conditions. The culture may be any of stationary, shake and aerial cultures but shake or aerial culture is advantageous. The culture preferably takes place at a temperature of 25 to 33°C, more preferably at approximately from 27 to 29°C. Preferably the pH of the medium should be maintained in a neutral range, from about 5.5 to about 8.5. The incubation period varies depending upon the composition of the medium, culture conditions such as temperature, etc. but is generally for about 2 to about 14 days. The culture is completed when the above described antibiotics reach their maximum titer.

The antibiotic product may be isolated and purified by conventional methods.

For isolation and purification of the substances having antibacterial activity, there is preferably adopted a method which after centrifuging or filtering the culture solution to remove cells, uses difference in dissolution property or solubility in appropriate solvents, difference in precipitation ability or precipitation rate from a solution, difference in adsorption affinity to various adsorbents, difference in distribution between two types of liquid phases, etc. These methods may be used singly, in combination in optional orders, or repeatedly, depending upon necessity.

Among the products obtainable by culturing the YS-02930 K strain, two types of substance (D and E substances) having antibacterial activity are separately spotted at Rf values of 0.58 and 0.62 when a mixture of these substances is chromatographed on Silica Gel 60 $F_{254}$ made by Merck Inc. using chloroform:methanol:28% ammonia water (40:10:0.2) as a developing solvent; utilizing this property, these substances may easily be isolated. A purified product isolated from the former spot is the D substance and the E substance is a purified product isolated from the latter spot.

The H substance may be isolated as a spot of an Rf value of 0.62 by thin layer chromatography, on Silica Gel 60 $F_{254}$ thin layer plate made by Merck Inc., using as a developing solvent chloroform:methanol:28% ammonia water (160:40:0.5).

(Products)

The physicochemical properties of the thus obtained antibiotics are as follows:

(A) Physicochemical Properties of the D Substance:

(1) Ultraviolet Absorption Spectrum: $\lambda_{max}^{MeOH}$ 283 nm

(2) Infrared Absorption Spectrum: The infrared absorption spectrum of the substance according to the potassium bromide tablet method is shown in Figure 1.

(3) Nuclear Magnetic Resonance Spectrum: Nuclear magnetic resonance spectrum of the substance in chloroform-d at 100 MHz is shown in Figure 2.

(4) Mass Spectrum:
Major fragment peaks by EI-MS: 158, 407 and 581 ($M^+$)
Peak of its TMS derivative by EI-MS: 797 ($M^+$)

(5) Appearance: colorless powder

(6) Basic, neutral or acidic: basic substance

(7) Rf values of thin layer chromatography:
Silica Gel 60 $F_{254}$ (made by Merck Inc.) was used:
Detection: UV at 254 nm.

| Developing Solvents | Rf Value |
|---|---|
| Chloroform:methanol (5:1) | 0.22 |
| Chloroform:methanol:28% ammonia water (40:10:0.2) | 0.58 |

(B) Physicochemical Properties of the E Substance:

(1) Ultraviolet absorption spectrum: $\lambda_{max}^{MeOH}$ 240 nm

(2) Mass Spectrum:
Major fragment peaks by EI-MS: 158, 423 and 597 ($M^+$)
Peak of its TMS derivative by EI-MS: 813 ($M^+$)
(3) Appearance: colorless powder
(4) Basic, neutral or acidic: basic substance
(5) Solubility: soluble in methanol, ethanol, acetone, ethyl acetate and chloroform
(6) Rf values of thin layer chromatography:
Silica Gel 60 $F_{254}$ (made by Merck Inc.) was used:
Detection: UV at 254 nm.

| Developing Solvents | Rf Value |
|---|---|
| Chloroform:methanol (5:1) | 0.25 |
| Chloroform:methanol:28% ammonia water (40:10:0.2) | 0.62 |

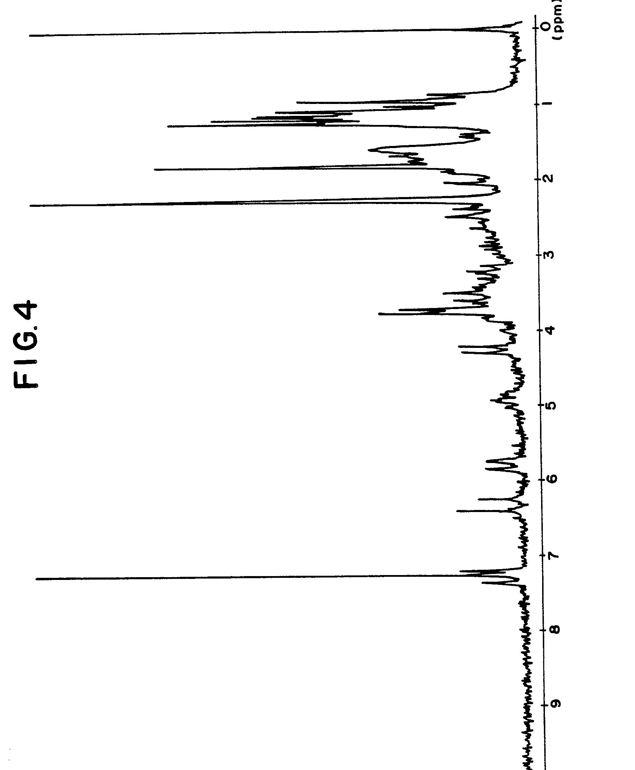

(C) Physicochemical Properties of the H Substance:
(1) Ultraviolet absorption spectrum: $\lambda_{max}^{MeOH}$ 283 nm
(2) Infrared Absorption Spectrum: The infrared absorption spectrum of this substance by the potassium bromide tablet method is shown in Figure 3.
(3) Nuclear Magnetic Resonance Spectrum:
The nuclear magnetic resonance spectrum of this substance in chloroform-d at 100 MHz is shown in Figure 4.
(4) Mass Spectrum:
Major fragment peaks by EI-MS: 158, 174, 379 and 553 ($M^+$)
(5) Molecular Weight and Molecular Formula: 553, $C_{30}H_{51}NO_8$
(6) Appearance: colorless powder
(7) Basic, neutral or acidic: basic substance
(8) Rf values of thin layer chromatography:
Silica Gel 60 $F_{254}$ (made by Merck Inc.) was used:
Detection: UV at 254 nm.

| Developing Solvents | Rf Value |
|---|---|
| Chloroform:methanol: 28% ammonia water (160:40:0.5) | 0.62 |

The D, E and H substances described above all exhibit antibacterial activity against pathogenic bacteria such as Gram-positive bacteria, Gram-negative bacteria, Mycoplasma, etc.

From the foregoing physicochemical properties, biological activities, etc., the products of the fermentation of the present invention have been identified, with the D substance being represented by formula:

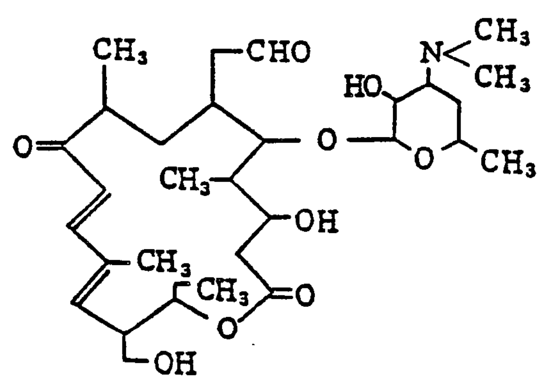

the E substance by formula:

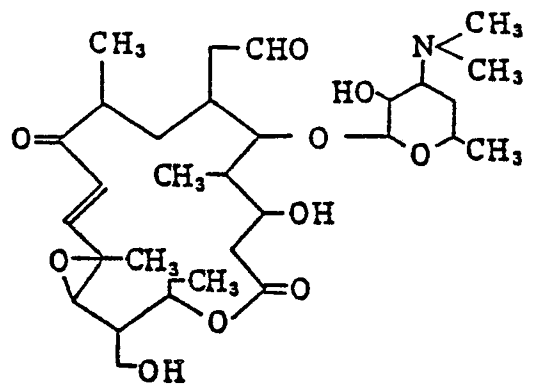

and the H substance by formula:

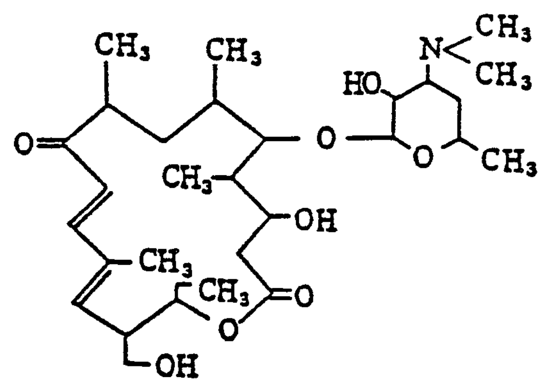

As described above, each of the antibiotics (D, E and H) provided by the process of the present invention exhibits excellent antibacterial activity against pathogenic bacteria such as Gram-positive bacteria, Gram-negative bacteria, Mycoplasma, etc., as compared to conventional macrolide antibiotics, and the antibiotics are useful for prophylaxis and treatment of diseases infected with these bacteria (reference is directed to the disclosure of U.S. Patent 4,438,109 for further information on this utility).

The present invention provides marked effects from an industrial viewpoint in that useful antibiotics (D, E and H) which have been hitherto produced only by chemical modification of products obtained by fermentation, can be produced directly by the fermentation process.

The present invention is illustrated in more detail by the following Examples.

Example 1

60 ml aliquots of culture medium (pH 8.0) containing 2.0% white dextrin, 0.5% glucose, 0.5% polypeptone, 0.5% yeast extract, 0.52% brain heart infusion, 0.5% corn steep liquor, 0.3%, meat extract and 0.2% calcium carbonate were charged into 500 ml flasks, and sterilized at 120°C for 20 minutes. Hyphae of *Micromonospora* sp. YS-02930 K strain grown in Bennett's agar medium were scratched off and added to this medium and shaking culture was carried out at 27°C for 72 hours, to make a seed culture solution. Next, 3.0% of the seed culture solution was added to 25 liters of medium (pH 7.1) supplemented with 3.0% of potato starch, 1.5% of soybean meal, 0.5% of corn steep liquor, 0.2% of yeast extract, 0.05% of magnesium sulfate heptahydrate, 0.3% of sodium chloride, 0.002% of cobalt chloride hexahydrate and 0.03% of Adekanol (made by Asahi Denka Kogyo K.K.), which has been charged into a stainless-made fermentation tank of 30 liter volume. Incubation was continued for 72 hours at an aeration rate of 25 liters/min while agitating at 95 to 150 rotations/min at temperatures of 28.0 to 28.5°C, whereby antibacterial activity against *Bacillus subtilis* ATCC 6633 strain reached a maximum. Radiolite #600 (Showa Chemical Industry Co., Ltd.) was added to the thus obtained culture solution. After agitating the mixture, it was filtered to obtain 20 liters of the filtrate. After 0.1N sodium hydroxide was added to the filtrate to adjust pH to 8.5, 20 liters of ethyl acetate was added thereto followed by thorough agitation. After the ethyl acetate layer was separated, 5 liters of aqueous hydrochloric acid of pH 3 were added thereto followed by thorough agitation.

After separating the aqueous hydrochloric acid layer of pH 3, sodium bicarbonate was added to adjust the pH to 8.5. Then, 5 liters of ethyl acetate were added to the system followed by thorough agitation. The ethyl acetate layer was separated and anhydrous sodium sulfate was added thereto for dehydration. The sodium sulfate was separated by filtration. The ethyl acetate layer was then concentrated under reduced pressure to obtain 200 mg of a pale yellow substance.

Example 2

After 200 mg of the pale yellow substance obtained in Example 1 was dissolved in a small quantity of methanol, the solution was applied to a thin layer plate of Silica Gel $F_{254}$ manufactured by Merck Inc. in a belt shape, which was chromatographed using as a developing solvent chloroform:methanol:28% ammonia water (40:10:0.2). Fractions of an Rf value of 0.58 and exhibiting antibacterial activity against *Bacillus subtilis* ATCC 6633 strain were scratched off. The scratched-off silica gel powders were packed in a column to elute a substance having an anti-bacterial activity with chloroform:methanol:28% ammonia water (40:10:0.2). Thereafter the eluate was concentrated under reduced pressure to obtain 15 mg of pure antibiotic YS-02930 K-D as colorless powders.

Example 3

After 200 mg of the pale yellow substance obtained in Example 1 was dissolved in a small quantity of methanol, the solution was applied to a thin layer plate of Silica Gel 60 $F_{254}$ manufactured by Merck Inc. in a belt shape, which was chromatographed using as a developing solvent chloroform:methanol:28% ammonia water (40:10:0.2). Fractions of an Rf value of 0.62 and exhibiting antibacterial activity against *Bacillus subtilis* ATCC 6633 strain were scratched off. The scratched-off silica gel powders were packed in a column to elute a substance having an anti-bacterial activity with chloroform:methanol:28% ammonia water (40:10:0.2). Thereafter the eluate was concentrated under reduced pressure to obtain 10 mg of pure antibiotic YS-02930 K-E as colorless powder.

Example 4

Hyphae of Micromonospora sp. YS-02930 K strain grown in Bennett agar medium were scratched off and added to 60 ml aliquots of medium (pH 8.0) in 500 ml flasks. The medium contained 2.0% white dextrin, 0.5% glucose, 0.5% polypeptone, 0.5% yeast extract, 0.52% brain heart infusion, 0.5% corn steep liquor, 0.3% meat extract and 0.2% calcium carbonate and had been sterilized at 120°C for 20 minutes. Shaking culture was carried out at 27°C for 72 hours, to make a seed culture solution. Next, 3.0% of the seed culture solution was added to 100 liters of medium (pH 7.1) supplemented with 3.0% of potato starch, 1.5% of soybean mean, 0.5% of corn steep liquor, 0.2% of yeast extract, 0.05% of magnesium sulfate heptahydrate, 0.3% of sodium chloride, 0.002% of cobalt chloride hexahydrate and 0.03% of Adekanol (made by Asahi Denka Kogyo K.K.), which had been charged into a stainless-made fermentation tank of 150 liter volume. Incubation was continued for 72 hours at an aeration rate of 100 liters/min while agitating at 95 to 150 rotations/min at temperatures of 28.0 to 28.5°C, whereby antibacterial activity against Bacillus subtilis ATCC 6633 strain reached a maximum. Radiolite #600 (Showa Chemical Industry Co., Ltd.) was added to the thus obtained culture solution. After agitating the mixture, it was filtered to obtain 85 liters of the filtrate. After 0.1N sodium hydroxide was added to the filtrate to adjust pH to 8.5, 85 liters of ethyl acetate was added thereto followed by thorough agitation. After the ethyl acetate layer was separated, 10 liters of aqueous hydrochloric acid of pH 3 were added thereto followed by thorough agitation. After separating the aqueous hydrochloric acid layer of pH 3, sodium bicarbonate was added to adjust the pH to 8.5 Then, 10 liters of ethyl acetate were added to the system followed by thorough agitation. The ethyl acetate layer was separated and anhydrous sodium sulfate was added thereto followed by dehydration. Then anhydrous sodium sulfate was separated by filtration. The ethyl acetate layer was then concentrated under reduced pressure to obtain 800 mg of a pale yellow substance.

Example 5

800 mg of the pale yellow substance obtained in Example 4 was dissolved in 1 ml of a solution of chloroform:methanol:28% ammonia water (50:1:0.1). Then, 1 ml of the solution was charged to a column packed with 16 g of Wako Gel C-200 (made by Wako Junyaku K.K.), and chromatographed using as a developing solvent chloroform:methanol:28% ammoniua water (50:1:0.1); each 5 ml fraction was chromatographed using silica gel 60 F 254 (made by Merck Inc.) and chloroform:methanol:28% ammonia water (160:40:0.5) as a developing solvent. Fractions showing an Rf value of 0.62 in a detector with UV rays at 254 nm and exhibiting antibacterial activity against *Bacillus subtilis* ATCC 6633 strain were collected. The collected fractions were concentrated under reduced pressure to obtain 20 mg of pure antibiotic YS-02930 K-H as colorless powders. As indicated above, in the accompanying drawings, Figure 1 shows the IR spectrum of YS-02930 K-D and Figure 2 its NMR spectrum; and Figure 3 shows the IR spectrum of YS-02930 K-H and Figure 4 its NMR spectrum.

**Claims**

1. Microorganisms belonging to the species *Micromonospora* YS-02930 capable of producing at least one antibiotic of the formula:

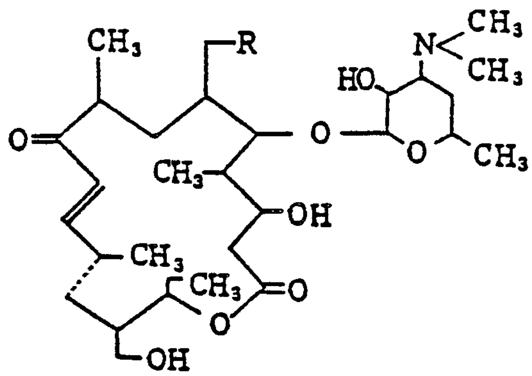

wherein R represents a hydrogen atom or a formyl group and the dotted line represents a double bond or

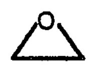

2. A microorganisms of the species according to claim 1 which is the type strain *Micromonospora* sp YS-02930 K (deposited with FRI Japan under the accession numbers FERM-P No. 7961 and FERM-BP No. 1076).

3. A microorganism of the genus *Micromonospora* which is a mutant or derivative of the microorganism according to claim 2.

4. A process for producing at least one antibiotic of the formula:

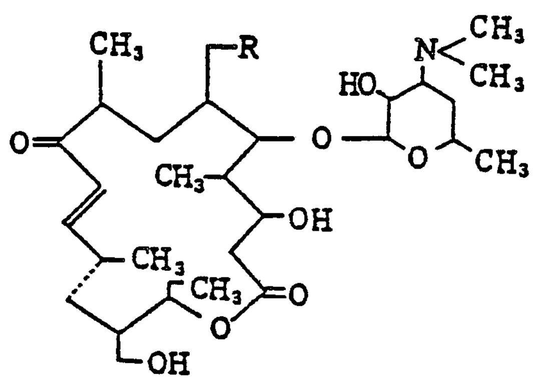

wherein R represents a hydrogen atom or a formyl group and the dotted line represents a double bond or

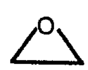

which comprises culturing microorganisms according to any of claims 1 to 3 and harvesting the resulting antibiotic(s) from the culture broth.

**Patentansprüche**

1. Mikroorganismus, der zur Art Micromonospora YS-02930 gehört und imstande ist, mindestens ein Antibiotikum der Formel

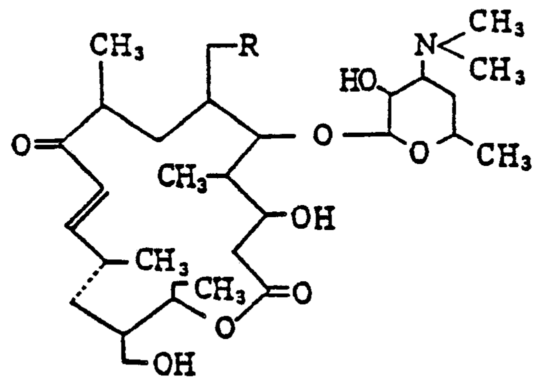

in der R ein Wasserstoffatom oder eine Formylgruppe bedeutet und die punktierte Linie eine Doppelbindung oder

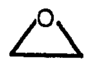

bedeutet, zu erzeugen.

2. Mikroorganismus der in Anspruch 1 angegebenen Art vom Stamm Micromonospora sp. YS-02930 K (hinterlegt bei FRI Japan under den Hinterlegungsnummern FERM-P Nr. 7961 und FERM-BP Nr. 1076).

3. Mikroorganismus der Art Mikromonospora, der eine Mutante oder ein Derivat des Mikroorganismus nach Anspruch 2 ist.

4. Verfahren zur Herstellung mindestens eines Antibiotikums der Formel

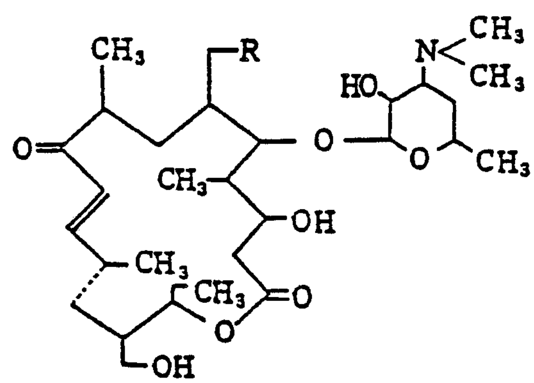

in der R ein Wasserstoffatom oder eine Formylgruppe bedeutet und die punktierte Linie eine Doppelbindung oder

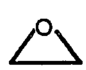

bedeutet, umfassend die Züchtung von Mikroorganismen nach einem der Ansprüche 1 bis 3, und Gewinnung des erhaltenen Antibiotikum (der erhaltenen Antibiotika) aus der Zellbrühe.

**Revendications**

1. Microorganismes appartenant à l'espèce *Micromonospora* YS-02930, capables de produire au moins un antibiotique de formule:

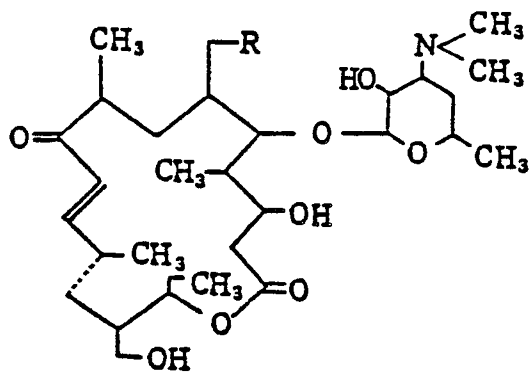

dans laquelle: R représente un atome d'hydrogène ou un groupe formyle; et le trait pointillé représente une double liaison ou

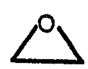

2. Microorganismes de l'espèce conforme à la revendication 1, qui est la souche type *Micromonospora* sp. YS-02930 K (déposée auprès du FRI du Japon sous les numéros d'identification FERM-P No. 7961 et FERM-BP No. 1076).

3. Microorganisme du genre *Micromonospora* qui est un mutant ou un dérivé du microorganisme conforme à la revendication 2.

4. Procédé de fabrication d'au moins un antibiotique de formule:

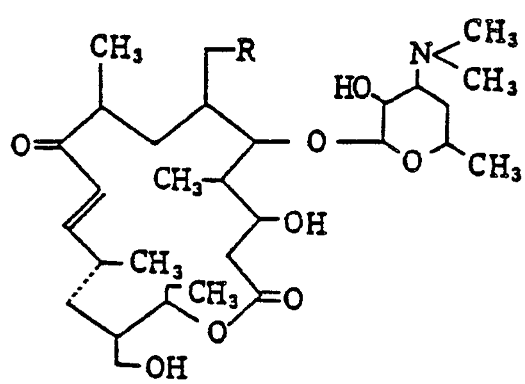

dans laquelle: R représente un atome d'hydrogène ou un groupe formyle; et le trait pointillé représente une double liaison ou

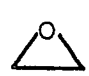

qui consiste à cultiver des microorganismes selon l'une des revendications 1 à 3, et à récolter le (ou les) antibiotique(s) résultant(s) à partir du bouillon de culture.

FIG. I

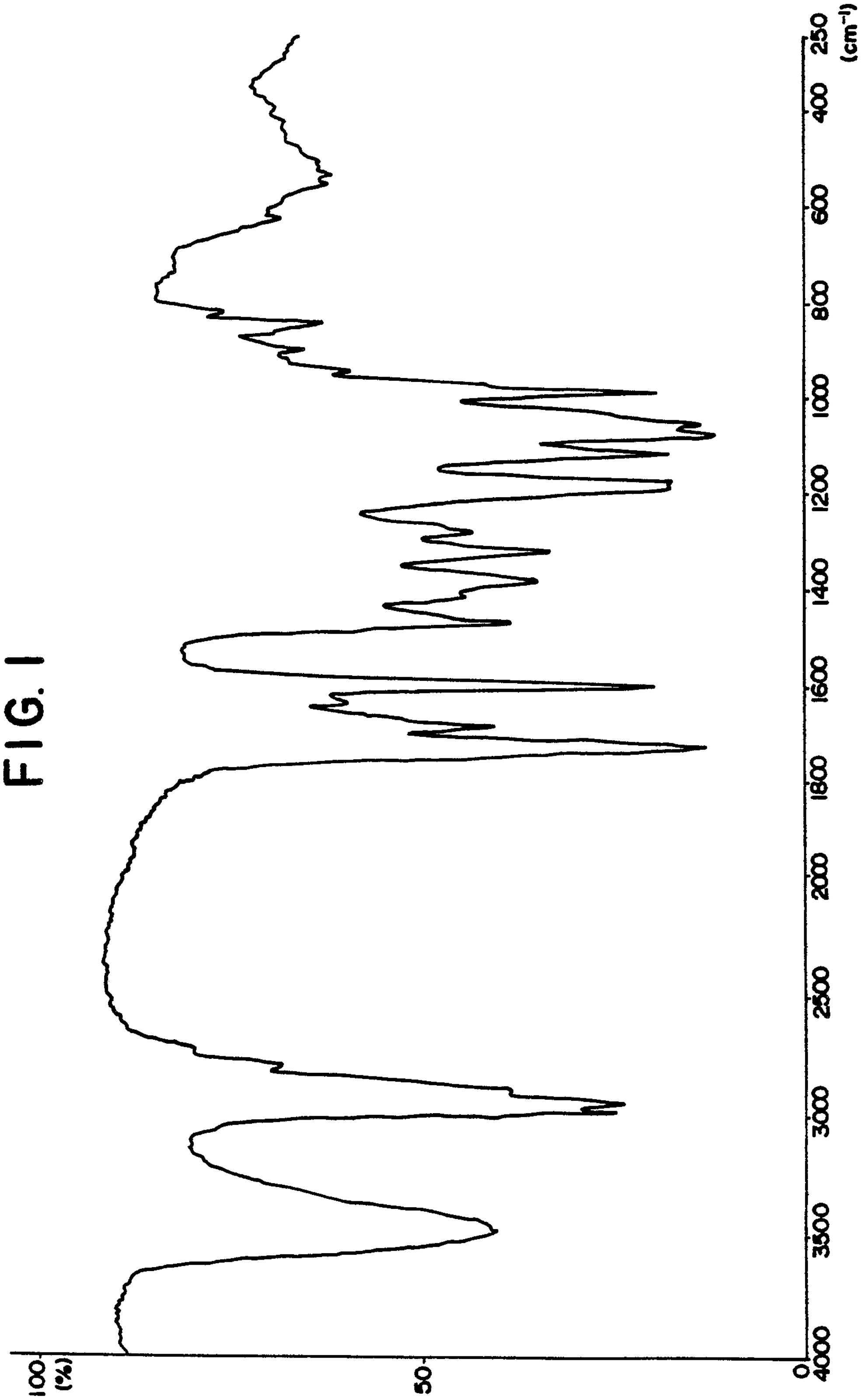

100
(%)
50
0
4000
3500
3000
2500
2000
1800
1600
1400
1200
1000
800
600
400
250
(cm⁻¹)

FIG. 2

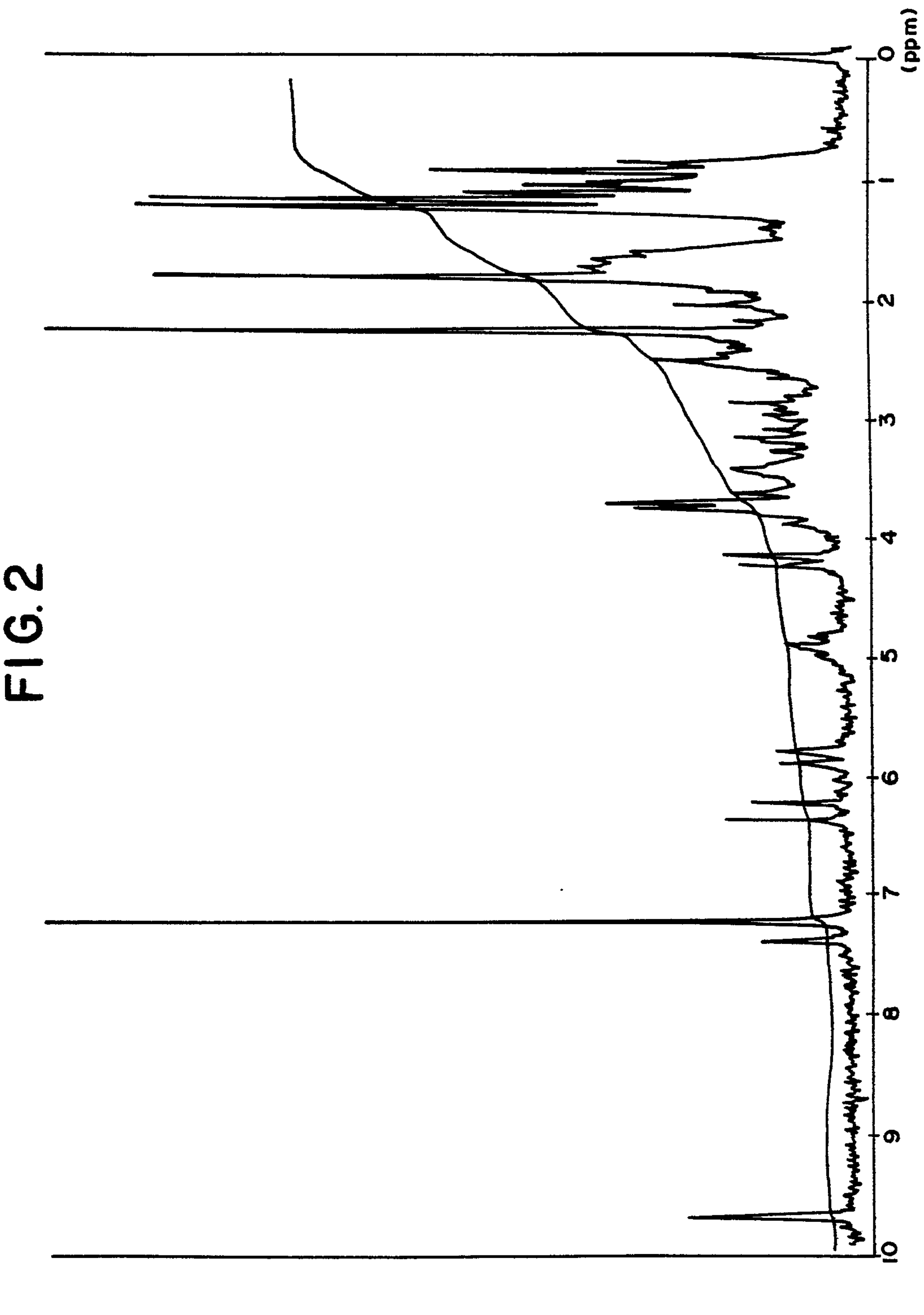

FIG. 3

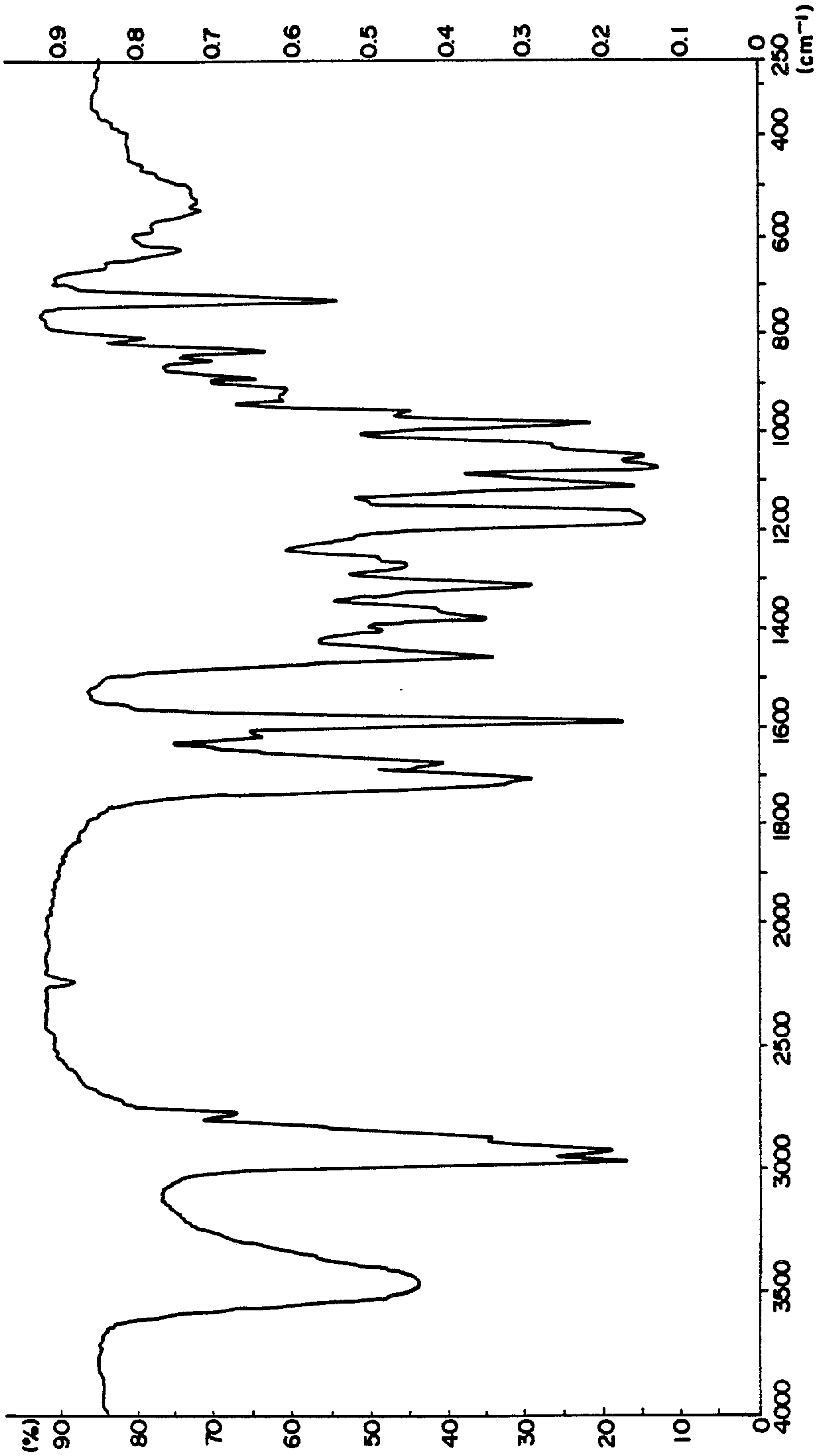
(%)
90
80
70
60
50
40
30
20
10
0
0.9
0.8
0.7
0.6
0.5
0.4
0.3
0.2
0.1
0
4000
3500
3000
2500
2000
1800
1600
1400
1200
1000
800
600
400
250
(cm⁻¹)

FIG.4